# FASCICULE DE BREVET EUROPEEN

(11) **EP 1 354 875 B1**
(45) Date de publication et mention de la délivrance du brevet: **24.11.2004**
(21) Numéro de dépôt: 03291157.0
(22) Date de dépôt: 19.05.2003
(51) Int. Cl.: C07D 209/42, C07K 5/02, C07K 5/06

(54) **Nouveau procédé de synthèse de l'acide (2S, 3aS, 7aS)-perhydroindole-2-carboxilique et de ses esters, et application à la synthèse du perindopril**
Verfahren zur Synthese von (2S,3aS,7aS)-Perhydroindol-2-carbonsäure und seiner Estern, und Verwendung in der Synthese von Perindopril
Method for synthesis of (2S,3aS,7aS)-perhydroindole-2-carboxylic acid and esters thereof; and use in the synthesis of perindopril

(43) Date de publication de la demande: 22.10.2003
(73) Titulaire: Les Laboratoires Servier, 92415 Courbevoie Cedex (FR)
(72) Inventeur: Dubuffet, Thierry, 76210 Bolbec (FR); Lecouve, Jean-Pierre, 76600 Le Havre (FR)

(56) Documents cités:
- EP-A- 0 308 339
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020

## Description

La présente invention concerne un procédé de synthèse de l'acide (2S, 3aS, 7aS)-perhydroindole-2-carboxylique et de ses esters, et leur application à la synthèse industrielle du perindopril et de ses sels pharmaceutiquement acceptables.

Plus spécifiquement, la présente invention concerne un nouveau procédé de synthèse des dérivés de formule (I) : 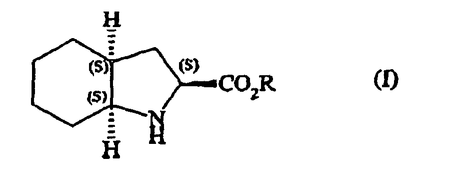 dans laquelle R représente un atome d'hydrogène ou un groupement benzyle ou alkyle (C₁-C₆) linéaire ou ramifié,
ainsi que leurs sels d'addition à un acide ou à une base minéral(e) ou organique.

Les composés de formule (I) obtenus selon le procédé de l'invention sont utiles dans la synthèse du perindopril de formule (II): 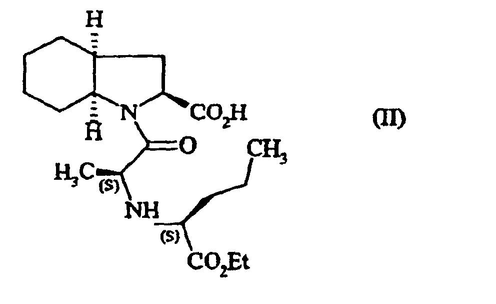 ainsi que dans celle de ses sels pharmaceutiquement acceptables.

Le perindopril, ainsi que ses sels, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse performant, permettant l'obtention sélective du diastéréoisomère (S,S,S) avec un bon rendement et une excellente pureté, à partir de matières premières bon marché.

Quelques méthodes de préparation des composés de formule (I) sont déjà connues.

Ainsi, le brevet EP 0 037 231 utilise comme matière première l'acide indole 2-carboxylique, qui est soumis à une hydrogénation catalytique sur rhodium pour donner un mélange des deux isomères cis endo de configurations respectives (2S, 3aS, 7aS) et (2R, 3aR, 7aR). Ce mélange est ensuite séparé de façon particulièrement laborieuse: synthèse du dérivé N-benzoylé, cristallisation fractionnée du sel du diastéréoisomère avec la (S)-α-phényl-éthylamine, libération des deux dérivés (S, S, S) et (R, R, R) N-benzoylés, puis élimination du groupement benzoyle, suivie d'un passage sur colonne échangeuse d'ions et d'une recristallisation.

Le brevet EP 0 115 345, pour cette même séparation, utilise plusieurs étapes nécessitant l'estérification de la fonction acide carboxylique par l'alcool benzylique, la salification de l'amino ester par la N-benzyloxycarbonyl-(S)-phénylalanine, la séparation par cristallisation fractionnée de l'isomère (S, S, S), la libération de la fonction aminée optionnellement suivie de la libération du groupement acide carboxylique.

Les brevets EP 0 308 339 et EP 0 308 341 utilisent également comme matière première l'acide indole 2-carboxylique, qui est dans un premier temps réduit en acide indoline 2-carboxylique, pour donner un mélange d'acide indoline carboxylique 2R et 2S, lesquels sont ensuite séparés par cristallisation fractionnée. L'isomère 2S est ensuite soumis à hydrogénation catalytique pour conduire au composé de formule (I).

La demanderesse a présentement mis au point un nouveau procédé de synthèse des dérivés de formule (I), à partir d'une matière première particulièrement bon marché, et qui permet l'obtention sélective du diastérèoisomère (S, S, S) avec un bon rendement et une excellente pureté.

Plus spécifiquement, la présente invention concerne un procédé de synthèse des composés de formule (I), caractérisé en ce que l'on condense l'acide (2-oxocyclohexyl)-acétique de formule (III): 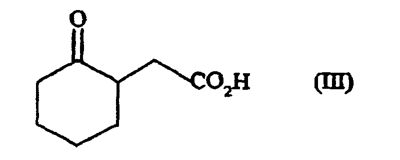 avec le (S)-phénylglycinol de formule (IV): 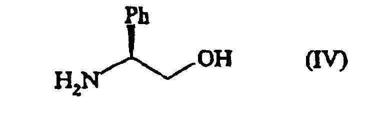 dans laquelle Ph représente le groupement phényle,
pour conduire au lactame tricyclique de formule (V) sous la forme d'un seul diastéréoisomère : 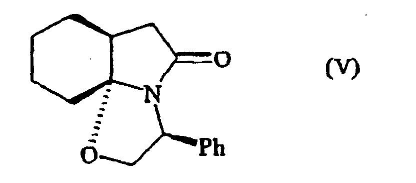 dans laquelle Ph représente le groupement phényle,
que l'on soumet à un agent réducteur en présence d'un acide de Lewis, pour conduire au composé de formule (VI): 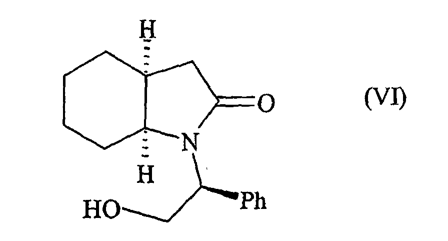 dans laquelle Ph représente le groupement phényle,
dont on clive la fonction 2-hydroxy-1-phényl-éthyle, pour conduire au composé de formule (VII) : 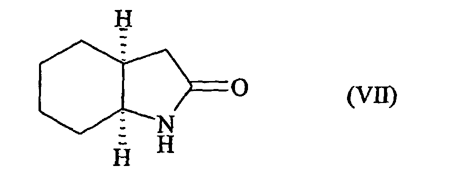 que l'on met en réaction, soit avec l'anhydride triflique, soit avec un réactif d'alkylation, pour conduire au composé de formule (VIII): 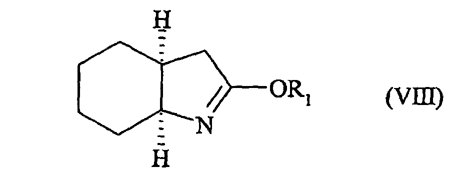 dans laquelle R₁ représente un groupement trifluorométhanesulfonate ou alkyle (C₁-C₆) linéaire ou ramifié,
que l'on met en réaction avec un réactif de cyanation, pour conduire au composé de formule (IX) : 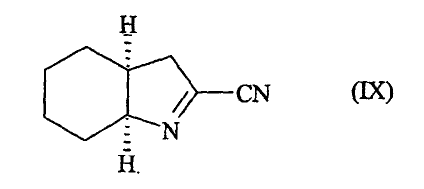 dont on hydrolyse la fonction nitrile en milieu acide aqueux ou alcoolique, pour conduire au composé de formule (X) : 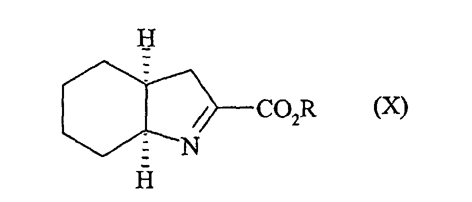 dans laquelle R est tel que défini dans la formule (I),
dont on réduit la double liaison par hydrogénation catalytique, pour conduire au composé de formule (I).

Les composés de formules (IX) et (X) sont des produits nouveaux, utiles comme intermédiaires de synthèse dans l'industrie chimique ou pharmaceutique, notamment dans la synthèse du composé de formule (I), et font à ce titre partie intégrante de l'invention.

Parmi les agents réducteurs utilisables pour la réduction du lactame de formule (V) dans le procédé de la présente invention, on peut citer à titre non limitatif le triéthylsilane.

Parmi les acides de Lewis utilisables pour la réduction du lactame de formule (V) dans le procédé de la présente invention, on peut citer à titre non limitatif l'éthérate du trifluorure de bore et le tétrachlorure de titane. L'acide de Lewis préféré est l'éthérate du trifluorure de bore.

Parmi les réactifs d'alkylation utilisables dans le procédé de la présente invention, on peut citer à titre non limitatif le sulfate de diméthyle, le sulfate de diéthyle et le tétrafluoroborate de triéthyloxonium.

Parmi les réactifs de cyanation utilisables dans le procédé de la présente invention, on peut citer à titre non limitatif le cyanure de triméthylsilyle, le cyanure de lithium, le cyanure de sodium et le cyanure de tétrabutylammonium. Le réactif de cyanation préféré est le cyanure de lithium.

Le composé de formule (I) ainsi obtenu a une très bonne pureté chimique et énantiomérique, ce qui rend son emploi particulièrement avantageux dans la synthèse du perindopril de formule (II).

A titre d'illustration, le couplage du composé de formule (I) obtenu selon le procédé de l'invention avec le composé de formule (XI): 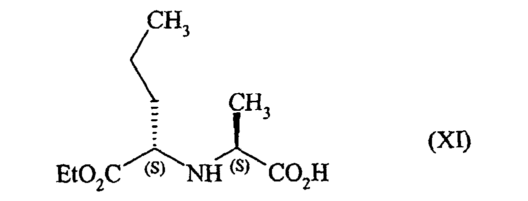 permet d'obtenir le perindopril de formule (II) avec une pureté et un rendement très satisfaisants.

### Définitions :

Par éther-couronne 12-C-4, on entend 1,4,7,10-tétraoxacyclododécane.

### EXEMPLE : Acide (2S,3aS,7aS)-perhydroindole-2-carboxylique, paratoluènesulfonate

### Stade A : (3S, 6aS, 10aR)-3-Phényloctahydro-5H-[1,3]oxazolo[2,3-i]indol-5-one :

Dans un réacteur, chauffer à reflux 200 g d'acide (2-oxocyclohexyl)-acétique, 175 g de (S)-phénylglycinol et 1 l de toluène en éliminant l'eau formée par entraînement azéotropique. Lorsqu'il ne décante plus d'eau, évaporer le toluène pour conduire à la (3S, 6aS, 10aR)-3-phényloctahydro-5H-[1,3]oxazolo[2,3-i]indol-5-one.

### Stade B : (3aS, 7aS)-1-[(1S)-2-Hydroxy-1-phényléthyl]-octahydro-2H-indol-2-one :

Dans un réacteur, chauffer à reflux pendant 15h 200 g du composé obtenu au stade précédent, 181 g de triéthylsilane, 664 g d'éthérate de trifluorure de bore et 1 l de dichlorométhane.
Après retour à température ambiante, le mélange réactionnel est versé sur une solution aqueuse glacée d'hydrogénocarbonate de sodium, puis le pH est amené à 6 par addition de soude. La phase organique est ensuite lavée, puis engagée dans le stade suivant.

### Stade C : (3aS, 7aS)-Octahydro-2H-indol-2-one :

Dans un réacteur, placer 200 g du composé obtenu au stade précédent, 1 l de dichlorométhane, puis amener le mélange à 0-5°C et ajouter lentement 92 g de chlorure de thionyle. Agiter ensuite 2 heures à reflux, puis , après retour à température ambiante, laver la phase organique par de l'eau puis la concentrer au tiers.
Le chlorure ainsi obtenu est ensuite réempâté sur silice, pour conduire par élimination d'acide chlorhydrique à l'énamide correspondant.
Celui-ci est ensuite hydrolysé par une solution aqueuse 5M d'acide sulfurique, pour conduire à la (3aS, 7aS)-octahydro-2H-indol-2-one.

### Stade D : (3aS, 7aS)-3a,4,5,6,7,7a-Hexahydro-3H-indole-2-carbonitrile :

Dans un réacteur, charger 200 g du composé obtenu au stade précédent, 403 g d'anhydride triflique, 185 g de diisopropyléthylamine et 1 l de dichlorométhane, puis agiter à température ambiante.
Après 16 heures de réaction, le mélange réactionnel est filtré et le filtrat mis à sec. Le produit brut est repris par du tétrahydrofurane. Cette solution est ajoutée à un mélange de 36 g de cyanure de lithium et de 9 g d'éther-couronne 12-C-4, puis agitée 2 heures supplémentaires à température ambiante.
Après addition d'eau, la phase organique est lavée, puis évaporée, pour conduire au (3aS, 7aS)-3a,4,5,6,7,7a-hexahydro-3H-indole-2-carbonitrile.

### Stade E : Acide (3aS, 7aS)-3a,4,5,6,7,7a-hexahydro-3H-indole-2-carboxylique :

Dans un réacteur, charger 200 g du composé obtenu au stade précédent et 2 l d'une solution aqueuse d'acide chlorhydrique à 20 %. Le milieu réactionnel est porté au reflux pendant 4 heures, puis extrait au dichlorométhane. Les phases organiques sont lavées, puis les solvants sont évaporés, pour conduire à l'acide (3aS, 7aS)-3a,4,5,6,7,7a-hexahydro-3H-indole-2-carboxylique.

### Stade F : Acide (2S,3aS,7aS)-perhydroindole-2-carboxylique, paratoluènesulfonale :

Dans un hydrogénateur, placer 200 g du composé obtenu dans le stade précédent, en solution dans l'acide acétique, puis 5 g de Pd/C à 10 %. Hydrogéner sous pression de 5 bars à température ambiante, jusqu'à absorption de la quantité théorique d'hydrogène. Eliminer le catalyseur par filtration, puis additionner un équivalent d'acide paratoluènesulfonique, agiter à 30°C 1h, refroidir entre 0 et 5°C et récolter le solide obtenu par filtration. Laver le gâteau et le sécher jusqu'à poids constant.
Le paratoluènesulfonate de l'acide (2S, 3aS, 7aS)-perhydroindole-2-carboxylique est ainsi obtenu avec une pureté énantiomérique de 99 %.

## Revendications

1. Procédé de synthèse des composés de formule (I): 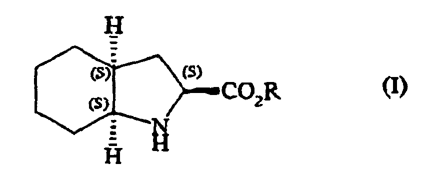 dans laquelle R représente un atome d'hydrogène ou un groupement benzyle ou alkyle (C₁-C₆) linéaire ou ramifié,
ainsi que leurs sels d'addition à un acide ou à une base minéral(e) ou organique,
**caractérisé en ce que** l'on condense l'acide (2-oxocyclohexyl)-acétique de formule (III) : 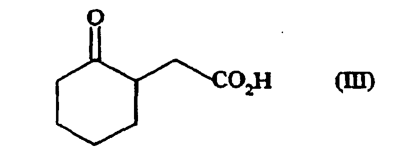 avec le (S)-phénylglycinol de formule (IV): 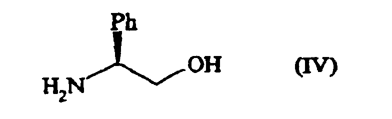 dans laquelle Ph représente le groupement phényle,
pour conduire au lactame tricyclique de formule (V) sous la forme d'un seul diastéréoisomère ; 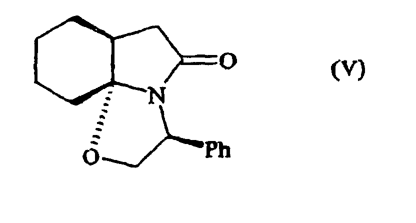 dans laquelle Ph représente le groupement phényle,
que l'on soumet à un agent réducteur en présence d'un acide de Lewis, pour conduire au composé de formule (VI): 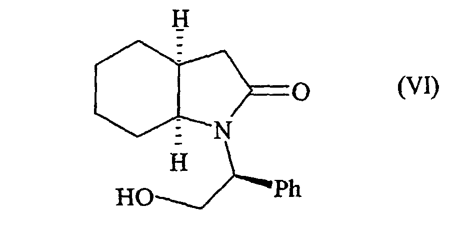 dans laquelle Ph représente le groupement phényle,
dont on clive la fonction 2-hydroxy-1-phényl-éthyle, pour conduire au composé de formule (VII) : 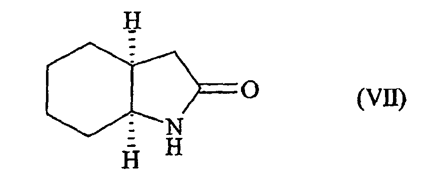 que l'on met en réaction, soit avec l'anhydride triflique, soit avec un réactif d'alkylation, pour conduire au composé de formule (VIII): 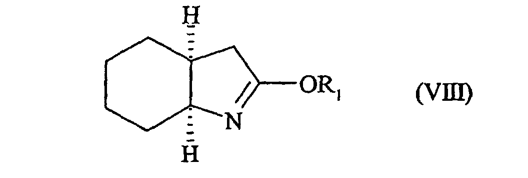 dans laquelle R₁ représente un groupement trifluorométhanesulfonate ou alkyle (C₁-C₆) linéaire ou ramifié,
que l'on met en réaction avec un réactif de cyanation, pour conduire au composé de formule (IX) : 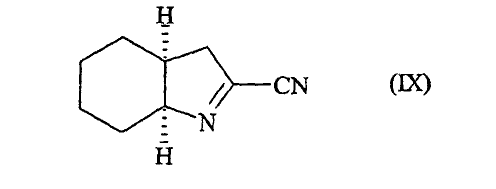 dont on hydrolyse la fonction nitrile en milieu acide aqueux ou alcoolique, pour conduire au composé de formule (X): 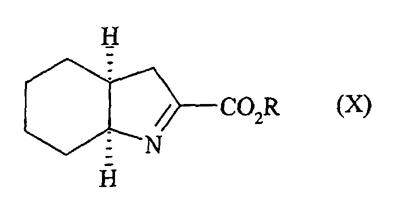 dans laquelle R est tel que défini dans la formule (I),
dont on réduit la double liaison par hydrogénation catalytique, pour conduire au composé de formule (I).

2. Procédé de synthèse selon la revendication 1, **caractérisé en ce que** l'agent réducteur est le triéthylsilane.

3. Procédé de synthèse selon l'une quelconque des revendications 1 ou 2, **caractérisé en ce que** l'acide de Lewis est l'éthérate du trifluorure de bore.

4. Procédé de synthèse selon l'une quelconque des revendications 1 à 3, **caractérisé en ce que** le réactif de cyanation est le cyanure de lithium.

5. Composé de formule (IX):

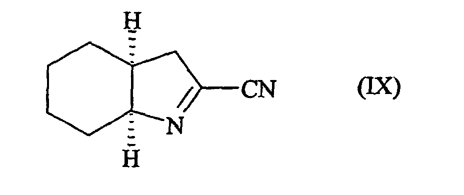

6. Composé de formule (X) : 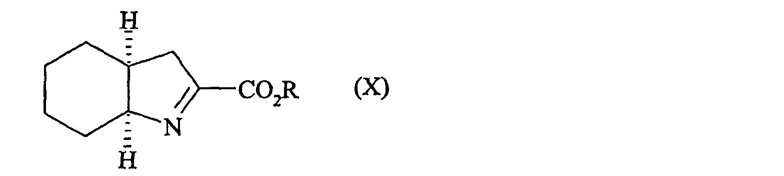 dans laquelle R représente un atome d'hydrogène ou un groupement benzyle ou alkyle (C₁-C₆) linéaire ou ramifié.

7. Procédé de synthèse selon l'une quelconque des revendications 1 à 4, permettant l'obtention du dérivé de formule (I) dans laquelle R représente un atome d'hydrogène.

8. Procédé de synthèse du perindopril ou de ses sels pharmaceutiquement acceptables à partir d'un composé de formule (I), **caractérisé en ce que** ledit composé de formule (I) est obtenu selon le procédé de la revendication 1.

## Patentansprüche

1. Verfahren zur Synthese der Verbindungen der Formel (I): 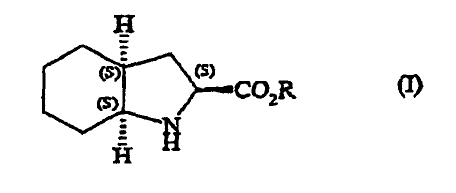 in der R ein Wasserstoffatom oder eine Benzylgruppe oder eine geradkettige oder verzweigte (C₁-C₆)-Alkylgruppe bedeutet,
sowie von deren Additionsalzen mit einer anorganischen oder organischen Säure, **dadurch gekennzeichnet, daß** man (2-Oxocyclohexyl)-essigsäure der Formel (III): 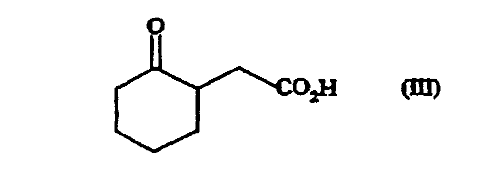 mit (S)-Phenylglycinol der Formel (IV): 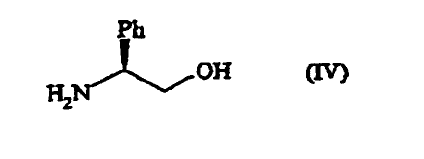 in der Ph die Phenylgruppe bedeutet, umsetzt,
zur Bildung des tricyclischen Lactams der Formel (V) in Form eines einzigen Diastereoisomeren: 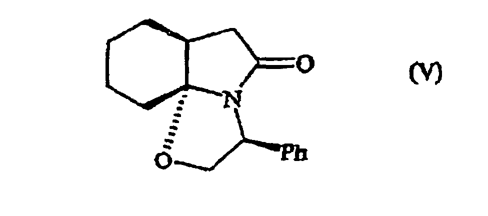 in der Ph die Phenylgruppe bedeutet,
welches man der Einwirkung eines Reduktionsmittels in Gegenwart einer Lewis-Säure unterwirft zur Bildung der Verbindung der Formel (VI): 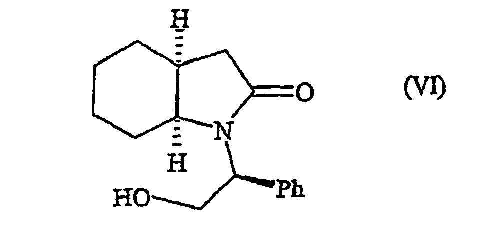 in der Ph die Phenylgruppe darstellt,
von welcher man die 2-Hydroxy-1-phenyl-ethyl-Gruppe abspaltet zur Bildung der Verbindung der Formel (VII): 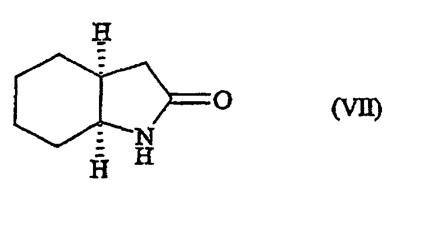 welche man entweder mit Trifluormethansulfonsäureanhydrid oder mit einem Alkylierungreagens umsetzt zur Bildung der Verbindung der Formel (VIII): 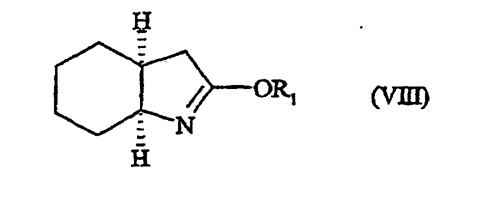 in der R₁ eine Trifluormethansulfonat-Gruppe oder eine geradkettige oder verzweigte (C₁-C₆)-Alkylgruppe darstellt,
welche man mit einem Cyanierungsreagens umsetzt zur Bildung der Verbindung der Formel (IX): 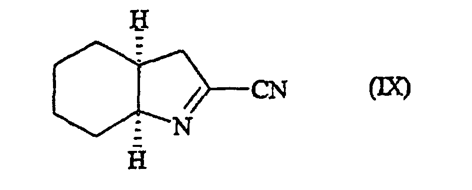 deren Nitrilfunktion man in wässrigem oder alkoholischem saurem Medium hydrolysiert zur Bildung der Verbindung der Formel (X): 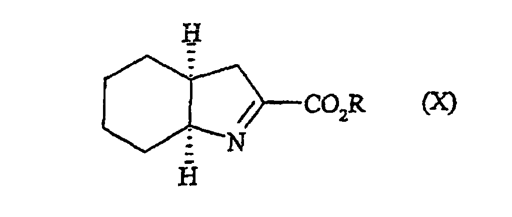 in der R die bezüglich der Formel (I) angegebenen Bedeutungen besitzt,
deren Doppelbindung man durch katalytische Hydrierung reduziert zur Bildung der Verbindung der Formel (1).

2. Syntheseverfahren nach Anspruch 1, **dadurch gekennzeichnet, daß** das Reduktionsmittel Triethylsilan ist.

3. Syntheseverfahren nach einem der Ansprüche 1 oder 2, **dadurch gekennzeichnet, daß** die Lewis-Säure Bortrifluorid-Etherat ist.

4. Syntheseverfahren nach einem der Ansprüche 1 bis 3, **dadurch gekennzeichnet, daß** das Cyanierungsreagens Lithiumcyanid ist.

5. Verbindung der Formel (IX):

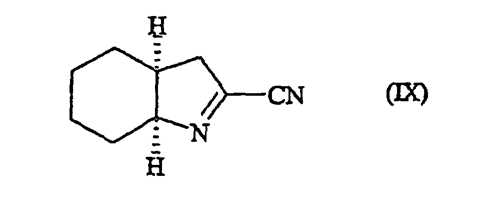

6. Verbindung der Formel (X): 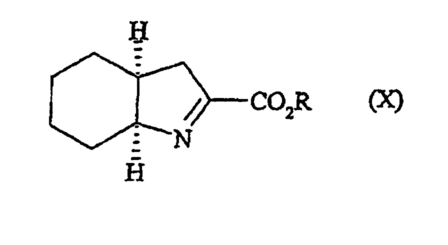 in der R ein Wasserstoffatom oder eine Benzylgruppe oder eine geradkettige oder verzweigte (C₁-C₆)-Alkylgruppe bedeutet.

7. Syntheseverfahren nach einem der Ansprüche 1 bis 4 für die Herstellung des Derivats der Formel (I), in der R ein Wasserstoffatom bedeutet.

8. Verfahren zur Synthese von Perindopril oder von seinen pharmazeutisch annehmbaren Salzen, ausgehend von einer Verbindung der Formel (I), **dadurch gekennzeichnet, daß** die verwendete Formel (I) nach dem Verfahren von Anspruch 1 hergestellt wird.

## Claims

1. Process for the synthesis of compounds of formula (I) : 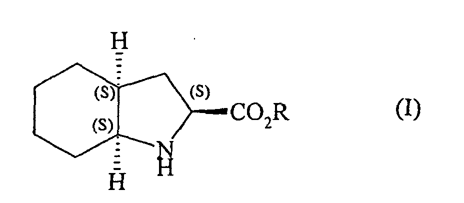 wherein R represents a hydrogen atom or a benzyl or linear or branched (C₁-C₆)alkyl group,
and addition salts thereof with a mineral or organic acid or base,
**characterised in that** (2-oxocyclohexyl)acetic acid of formula (III) : 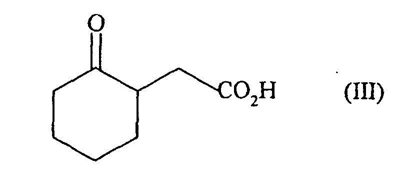 is condensed with (S)-phenylglycinol of formula (IV) : 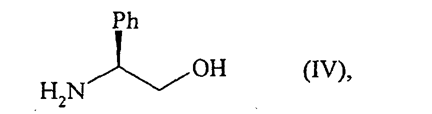 wherein Ph represents a phenyl group,
to yield the tricyclic lactam of formula (V) in the form of a single diastereoisomer : 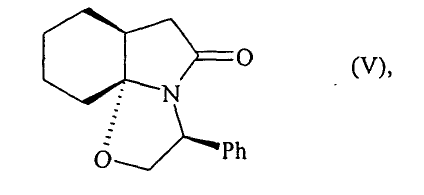 wherein Ph represents a phenyl group,
which is subjected to the action of a reducing agent in the presence of a Lewis acid to yield the compound of formula (VI) : 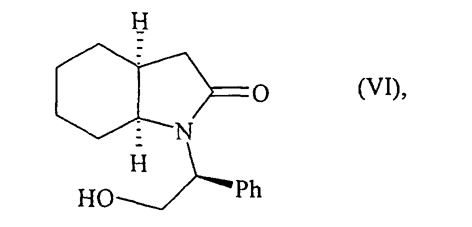 wherein Ph represents a phenyl group,
the 2-hydroxy-1-phenyl-ethyl function of which is cleaved to yield the compound of formula (VII) : 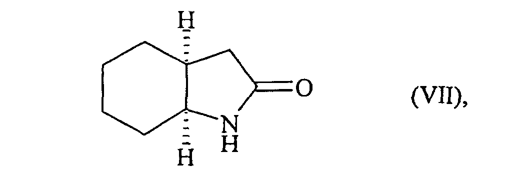 which is reacted either with triflic anhydride, or with an alkylation reagent, to yield a compound of formula (VIII) : 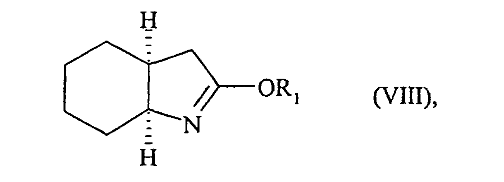 wherein R₁ represents a trifluoromethanesulphonate or linear or branched (C₁-C₆)alkyl group,
which is reacted with a cyanisation reagent to yield the compound of formula (IX) : 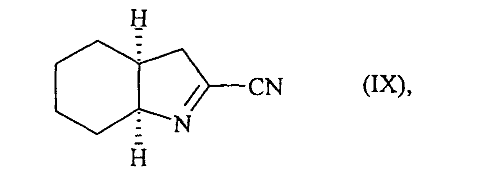 the nitrile function of which is hydrolysed in aqueous or alcoholic acidic medium to yield a compound of formula (X) : 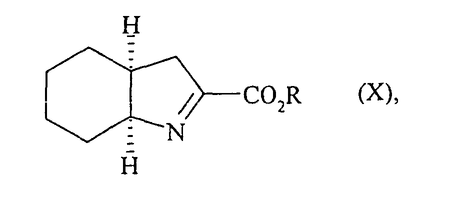 wherein R is as defined for formula (I),
the double bond of which is reduced by catalytic hydrogenation to yield a compound of formula (I).

2. Synthesis process according to claim 1, **characterised in that** the reducing agent is triethylsilane.

3. Synthesis process according to either claim 1 or claim 2, **characterised in that** the Lewis acid is boron trifluoride etherate.

4. Synthesis process according to any one of claims 1 to 3, **characterised in that** the cyanisation reagent is lithium cyanide.

5. Compound of formula (IX) :

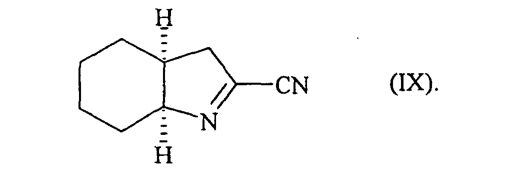

6. Compound of formula (X) : 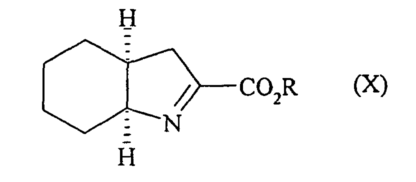 wherein R represents a hydrogen atom or a benzyl or linear or branched (C₁-C₆)alkyl group.

7. Synthesis process according to any one of claims 1 to 4, allowing the compound of formula (I) wherein R represents a hydrogen atom to be obtained.

8. Process for the synthesis of perindopril or pharmaceutically acceptable salts thereof starting from a compound of formula (I), **characterised in that** the said compound of formula (I) is obtained according to the process of claim 1.
